# EUROPEAN PATENT APPLICATION

(11) **EP 0 770 369 A1**
(43) Date of publication of application: **02.05.1997**
(21) Application number: 96307746.6
(22) Date of filing: 25.10.1996
(51) Int. Cl.: A61F 5/01

(54) **Ankle brace**

(30) Priority: 27.10.1995 US 8021; 04.10.1996 US 726444
(71) Applicant: Johnson & Johnson Professional, Inc., Raynham, Massachusetts 02767-0350 (US)
(72) Inventor: Fitzpatrick, Brian, Randolph, MA 02368 (US); Nunes, Victor M., Cumberland, RI 02864 (US)
(74) Representative: Fisher, Adrian John

(57) **Abstract**

There is disclosed an ankle brace having a cushion support comprising a bladder filled with a composite mixture of spherical objects and a lubricant.

## Description

### FIELD OF THE INVENTION

The present invention relates to an approved orthopedic device specifically an ankle brace for stabilizing the ankle after injury. The present ankle brace provides improved support for the ankle yet allows the needed flexibility for normal dorsiflexion and plantarflexion movement.

### BACKGROUND OF THE INVENTION

Various devices have been available for some time to provide management for injuries to the lower extremities such as fractures of the tibia and fibula, malleolar fractures or sever ankle sprains.

A common design of such devices is that shown in U.S. Patent 4,280,409. The device disclosed in this patent comprises a U-shaped stirrup member having a base portion and a pair of side wall portions which are attached to the base portion. The U-shaped stirrup member is constructed so that the heel of the wearers foot will fit into the stirrup. There are air inflatable liners or air bags on the interior of the side wall portions which can be inflated to engage the lateral and medial portions of the lower extremity of the patient. There are also some type of fasteners or straps that are provided to maintain the side wall portions and the air bags fitted around the leg of the patient. These straps are adjustable so that the same design of the ankle brace can be used for various patients.

Similar designs using air bladders are disclosed in U.S. Patents 5,125,400; 5,348,530; 5,445,602 and 4,977,891. Similar devices employing gel pads, which may have thermal properties in place of the air bags, are disclosed in U.S. Patent 4,964,402. There is disclosed in U.S. Patent 5,329,705 an orthopedic support device which includes an inner sole with an air bladder and a layer of foam material. U.S. Patent 4,628,945 also discloses an inflatable ankle brace of a similar design to that in the above mentioned U.S. Patent 4,280,489 which includes an air bag which contains a resilient compressible filler material. U.S. Patent 5,370,133 discloses a polyurethane molded boot which includes a cushion material of the same type used in the present invention as a compression pad in the walker.

The ankle braces of the type described above which include as a cushion material, an air bladder or a foam material or a combination of an air bladder and a foam material, have been found to be inadequate to provide the optimum support for the ankle in braces of the type described above. The air bladders or foam material have such flow characteristics that they do not provide even pressure to the ankle of the patient when the devices are worn. These air bladders or foam materials have a memory or a tendency to return or rebound to their original shape that they had prior to the application of pressure of fitting of the brace to the ankle or lower extremity.

### SUMMARY OF THE INVENTION

The present invention provides an improved lower extremity or ankle brace which provides a greater support for the patient during the natural gait cycle because the cushion material used in the present invention does not have memory characteristics or the desire to rebound to its original position The device provides minimal interference with plantarflexion and dorsiflexion during the normal walking gait of the patient.

The device of the present invention comprises a pair of rigid or semi-rigid shell members which may contain recesses which correspond to the general contour of the human ankle. The shells can be made of any semi-rigid material, preferably a thermoplastic material that is capable of being molded, such as, polypropylene, polyethylene, nylon, polycarbonate or acrylonitrile-butadiene-styrene (ABS). The preferred materials are the ABS type thermoplastic resin or polypropylene.

The semi-rigid shell may have a number of slots in the lower portion of the shell to provide a mechanism to secure a flexible material which would pass between the opposing shells and which would provide the stirrup for the device. Slots of this type are shown in U.S. Patent 4,964,402. In addition, additional slots may be formed in the upper portion of the device to provide openings to thread the fastening means which is used to fasten the device around the lower leg or calf. The preferred fastening means is a strap which may be a fabric strap which has on a portion of the ends of the strap a hook and loop fastener of the Velcro type commonly used to secure these devices to the leg of a patient.

The cushioning material that is used in the present invention comprises a bladder which contains a composite material comprising spherical objects and a lubricant. The bladder is secured to the inner, body contact, surfaces of the shell and is secured there by welding or by an adhesive or by hook and loop fasteners. The bladder itself is constructed of a polyurethane which is laminated to a brushed nylon fabric. The polyurethane provides a material which may be welded by radio frequency welding to the rigid support and the brush nylon fabric provides a superior surface against the ankle of the wearer. The bladder can be shaped by welding portions of the bladder in a pattern to provide a natural pocket for the malleoli of the patient. In addition, the welds will control the volume of the bladder in the particular location where control is desired, such as the top of the bladder.

As indicated above, the bladder itself is a composite mixture of spherical objects and a lubricant. This material is disclosed in U.S. Patent 5,421,874, which is incorporated herein by reference. The microspheres may be entirely spherical, oblong, egg-shaped, multi-sided, such as octagonal. The microspheres may be hollow or solid and if hollow, may have gaseous or liquid interiors. The spherical shapes preferred are generally plastic walled and gas filled microspheres. The diameter is generally less than 2000 microns in diameter and preferably in the range of 10 to 200 microns. The lubricant may be any lubricant selected from the group consisting of oils, greases, silicone-based lubricants, vegetable-based lubricants, petroleum-based lubricants, mineral-based lubricants, water-based lubricants, synthetic lubricants, mixtures of deionized water, propylene glycol, isoproterenol, polyethylene oxide and methyl parathion. The lubricant should be stable at temperatures between freezing and 120°F and should facilitate low friction siding and friction sliding and rolling contact of the microspheres to permit the microspheres to flow. The composite mixture has a specific gravity of from about 0.2 to about 0.5.

A significant characteristic of the present cushion or padding material is that it has no memory and once it is deformed it will maintain its configuration until additional force is applied to it. For that reason, in the particular application of an ankle brace, the material will conform to the body of the wearer when the brace is applied and once the brace is stabilized and the pressure of application is removed, the cushion material will not rebound and the brace will support the body of the patient in the position of the body when the brace was applied. This provides significant and greater support for the patient than if, for example, an air bladder or deformable foam is used as the cushion material.

In addition to the use of the microsphere composite cushion material in ankle braces, it may be used in other applications to pad bony prominence parts of the body and as comfort padding in other orthopedic applications. Such uses include, insoles, pillow crutch pads, cervical collars, strap padding, cast padding, therapeutic squeeze balls and padding in other bracing applications.

### BRIEF DESCRIPTION OF THE DRAWINGS

Fig. 1 is a side view of the brace of the present invention applied to the leg of a patient.

Fig. 2 is a perspective view of the brace of the present invention.

Fig. 3 is a view, partially, in section of the padding used in the brace of the present invention.

Fig. 4 is a partial cross section of the pad of Fig 3 viewed in the direction of the arrows in Fig. 3.

Fig. 5 shows a test device used to evaluate the force distribution between pads used in ankle braces.

Fig. 6 -10 are graphs that compare the load distribution forces in an air bladder with the bladder used in the brace ofthe present invention.

### DETAILED DESCRIPTION OF THE INVENTION

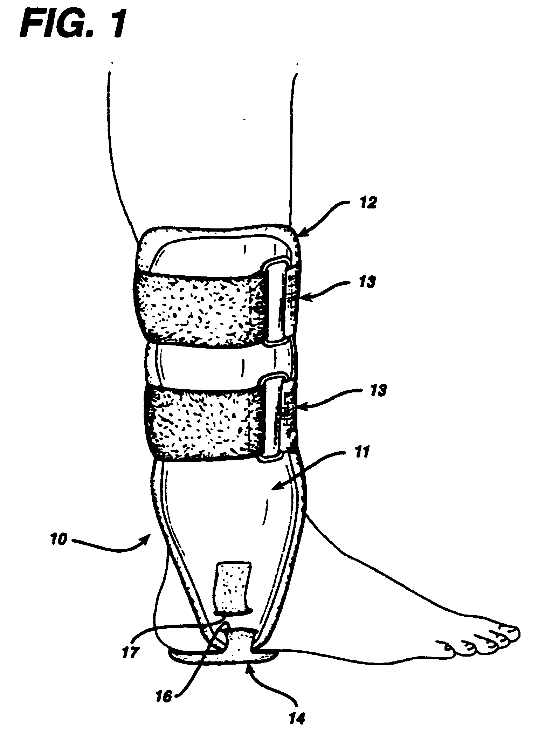

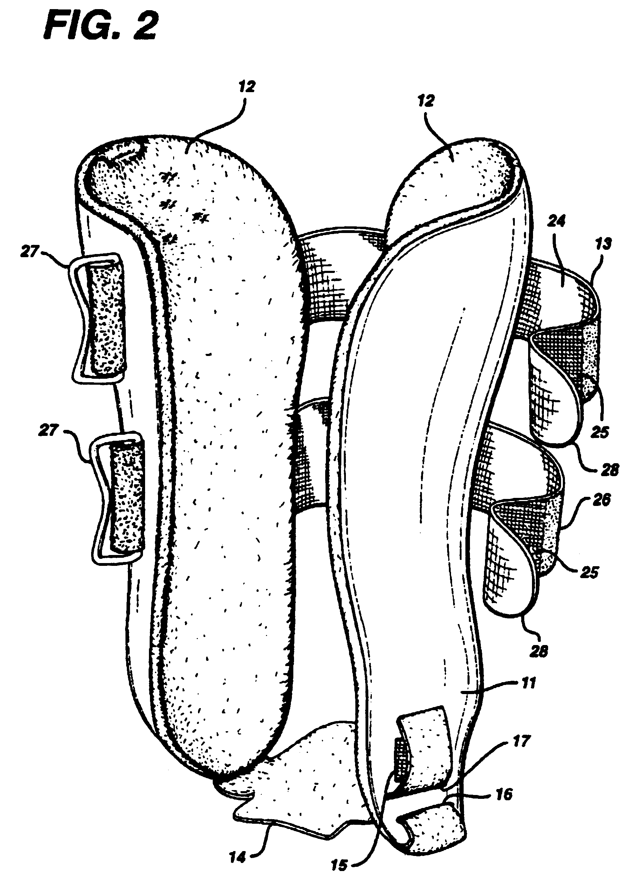

With reference to Fig. 1, the ankle brace 10 of the present invention includes a rigid sidewall shell 11 and a padding material 12 which is held to each side of the patient's lower limb by straps 13. The shells are held together by a stirrup 14 which is secured to the shell 11 by an adhesive or hook and eye fastener 15 which is shown in Fig. 2. The stirrup fits below the heel of the patient when the brace is worn.

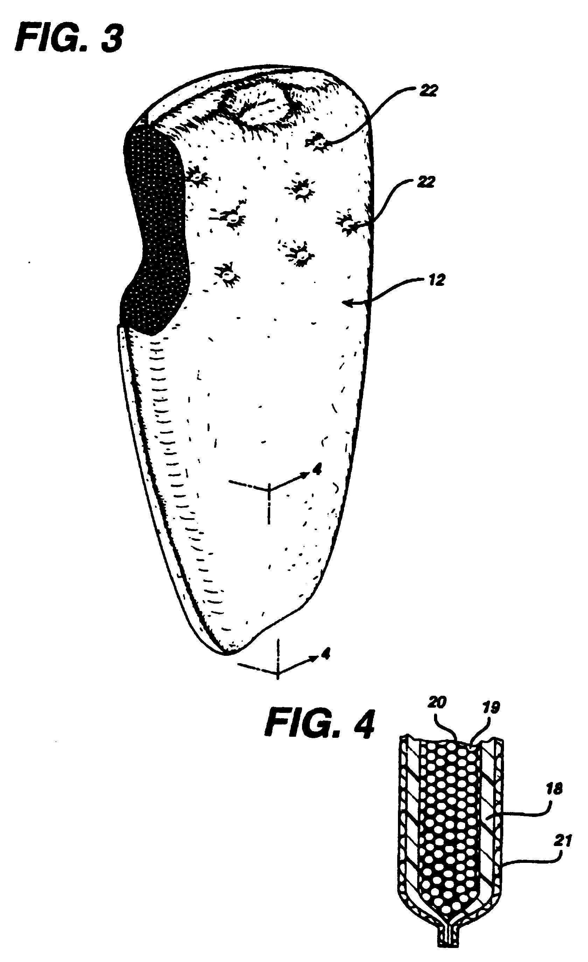

The shell 11 is made from a rigid or semi-rigid material, preferably one that is thermoplastic and therefore, capable of being easily molded to the desired configuration. Such plastic materials that are useful include polypropylene, polyethylene, nylon, polycarbonate and the preferred material an acrylonitrile-butadiene-styrene (ABS) resin. The stirrup material 14 can be a brushed nylon strap which can be threaded through slots 16 & 17 in the hard shell 11 and secured to the hard shell by adhesive or a hook and eye Velcro type fastener, 15. The padding material 12 of the ankle brace of the present invention is a material which is a thin bladder filled with microspheres and a lubricant. This material is fully described in U.S. Patent 5,421,874. This material is commercially available from TekSource, L.C. of Salt Lake City, Utah. The advantage of this material is that it has no memory and will provide much better support for the ankle then any material such as an air bladder or a foam which does have a memory. The microspheres within the bladder will flow only under shear and therefore will maintain their configuration and support of the tissue of the wearer of the brace until the brace is adjusted or removed. The padding material as shown in Fig. 3 and 4 comprises a polyurethane film 18 which contains the microspheres 19 which are surrounded by the lubricant 20. The outer surface of the bladder or padding 12 is a brushed nylon fabric 21 which provides comfort to the wearer. The bladder or pad 12 may be welded in different areas 22 to maintain the foam material in particular areas and to provide some configuration to the padding material.

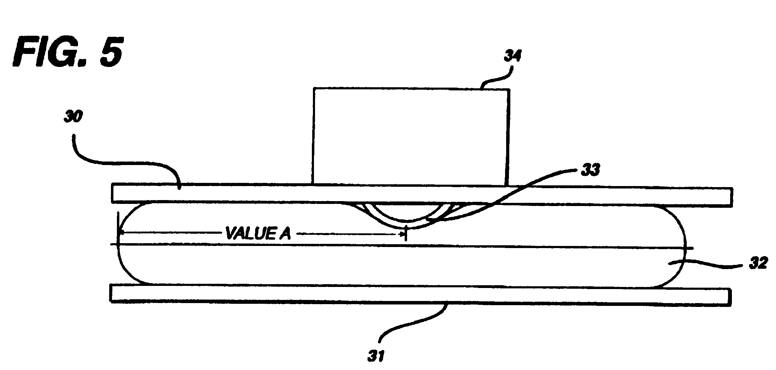

The straps 13 generally composed of lengths of nylon webbing 24 which have at their ends the hook structure 25 and the adjacent loop structure 26 which covers the webbing in various lengths to allow the adjustment of the straps to fit patients having different size legs. There are buckles 27 secured to one end of the strap 13. The straps are affixed by threading the end of the straps 28 through the buckle and turning the strap back on itself so that the hooks 25 can engage the loops 26 to secure the straps in position on the wearer. The padding or cushioning material used in the present invention is a significant improvement over the prior art air bladders or foam cushions because it provides an improved distribution of force when the brace is affixed to the patient, to support the patient's ankle. The force distribution characteristics of a cushion material can be determined by testing with the device shown in Fig. 5. The device consists of two rigid planar surfaces 30 and 31 between which the cushion material to be tested 32 is placed. There is a small piece of pipe, such as polyvinylchloride pipe 33 which is affixed to the bottom of the upper planar or surface 31. A weight 34, in this case about 2.664 kilograms, is placed on the top side of the upper planar surface over the PVC pipe. On the upper side of the lower planar surface is placed a sensor strip, Type 9800 which can record data using the TEK SCAN ISCAN1 Software. The sensor employed in the tests and evaluations of the materials was three inches by eight inches. The force was applied and 20 seconds of data were averaged to produce a data set for each run. In the following experiments, the distance, Value "A", was changed to determine the change in pressure with the changes in position of the PVC pipe. This reflects different positions of the malleoli in relation to the padding. In this test, the lower planar surface represents the ankle brace. The upper planar surface and the piece of PVC pipe represent the patient's ankle. The piece of PVC pipe represents the malleoli. The distribution of the force is significant because sufficient force must be applied to support the ankle but poorly distributed force will result in high pressure areas which could cause sores.

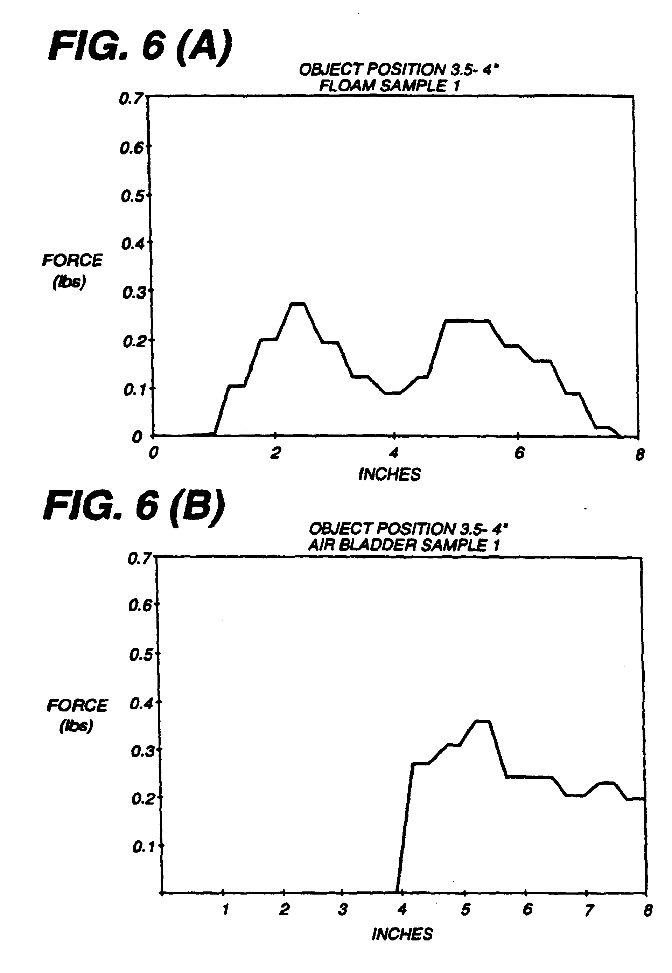

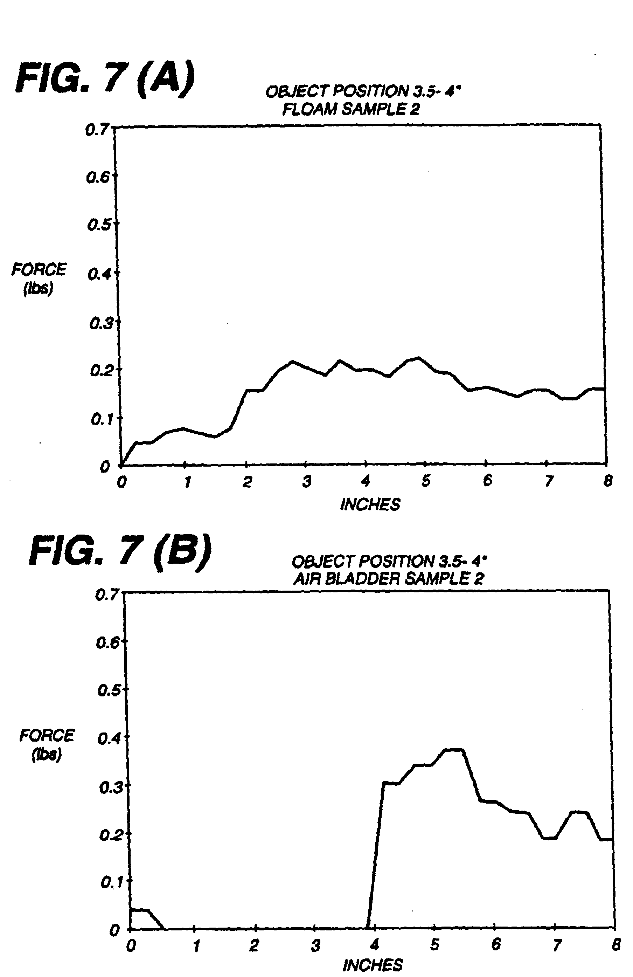

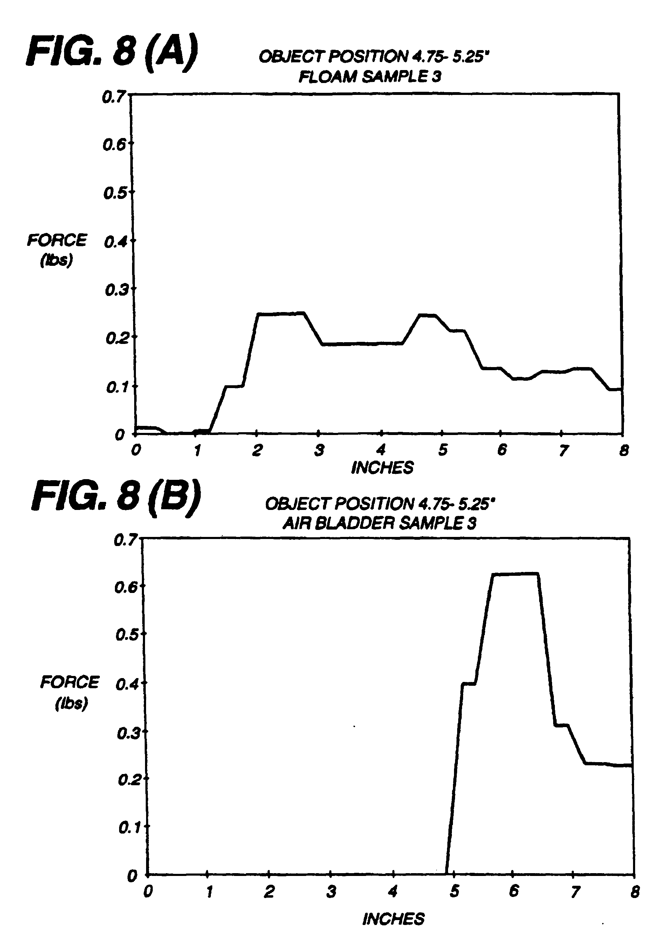

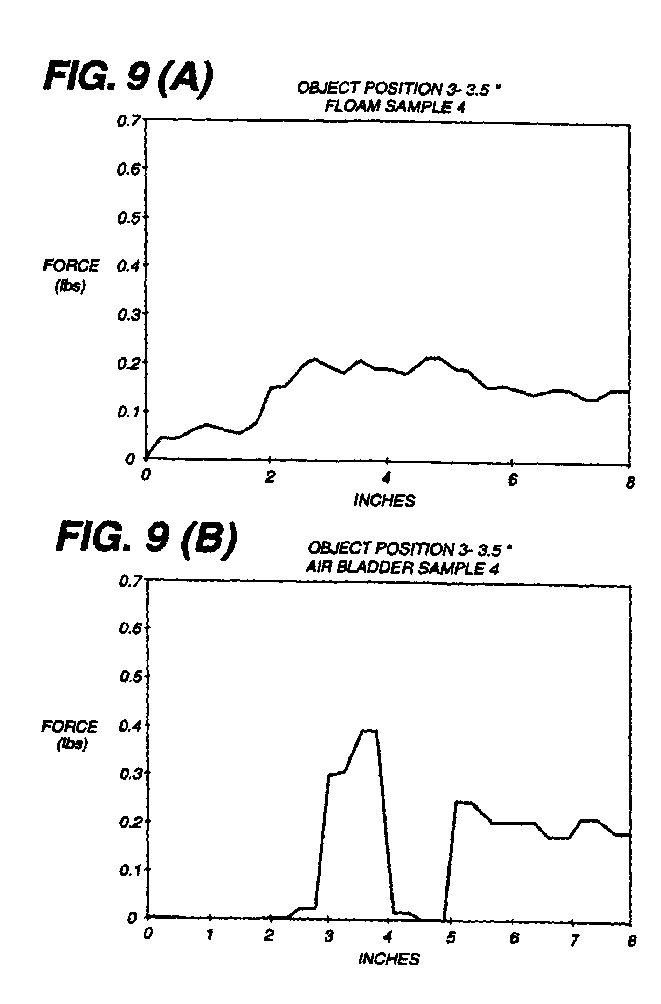

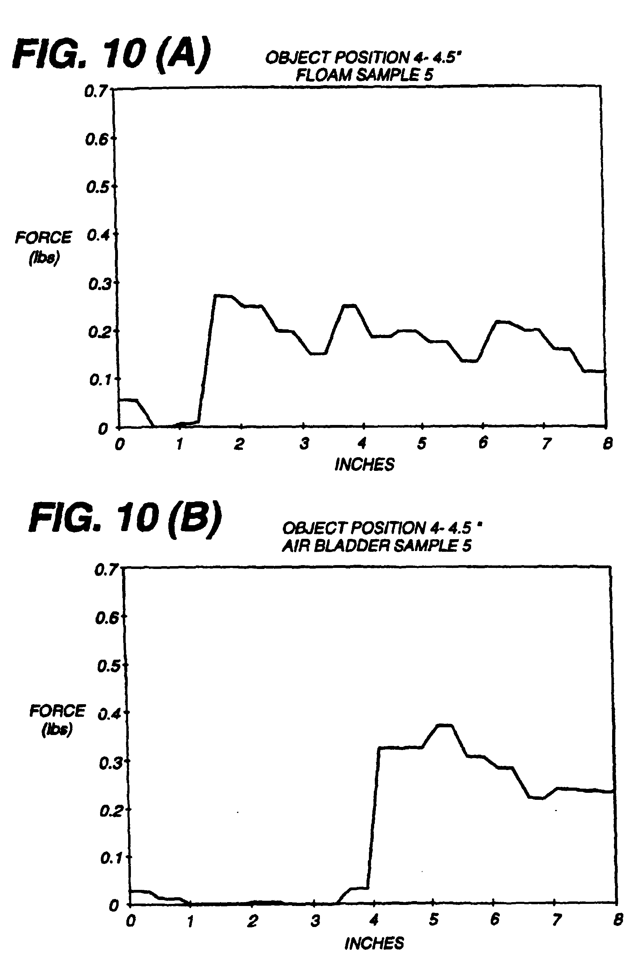

A number of different samples were run and in each case a sample using the cushion bladder of the present invention, which is identified as FLOAM™, and an air bladder taken from an AIRCAST™ brand ankle brace. The brace padding was placed on the lower planar surface on top of the sensor strip load cell. The upper planar surface with the PVC pipe was then in contact with the bladder. A weight of approximately 2.664 kilograms was placed above the PVC pipe. The weight is kept in place for approximately 20 seconds to record the forced distribution information which is plotted and shown in Fig. 6-10. The figures plot pair samples of the FLOAM™ padding of the present brace with an air bladder from an AIRCAST™ brace. It is very clear from the figures that the FLOAM™ samples distribute the force in a much more uniform manner than an air bladder. The following tables show the standard deviation of the data points as shown in the figures and the difference between the standard deviation is calculated and recorded as a percentage. The air bladder sample is considerably larger in standard deviation than the FLOAM™ sample employed in the brace of the present invention. This would indicate that the air bladder samples do not distribute the force as well as the FLOAM™ samples.

| Standard Deviation of Data Points | | | |
|---|---|---|---|
| Sample | FLOAM™ | Aircast | Aircast is larger by: |
| 1 | 0.0086 | 0.0128 | 149% |
| 2 | 0.0064 | 0.0134 | 208% |
| 3 | 0.0079 | 0.0191 | 240% |
| 4 | 0.0064 | 0.0119 | 185% |
| 5 | 0.0091 | 0.0134 | 147% |

The advantage of a more uniform distribution of force using the bladder of the present invention is that the support for the patients limb is significantly better because the force applied by the bladder is more uniformly distributed to the patient.

## Claims

1. In an ankle brace comprising a pair of spaced apart sidewall portions, a flexible support member affixed to each of the sidewall portions, said flexible members affixed to said sidewall portions so as to face one another, and fastening members to maintain the flexible members in contact with the wearer's ankle, the improvement comprising said flexible member comprising a flexible bladder containing a composite mixture of spherical objects and a lubricant which mixture will flow under the pressure of application of the brace to conform to the wearer's ankle and which mixture will cease to flow when the pressure is reduced so as to maintain its shape in contact with the wearer's ankle.

2. The brace of claim 1 in which the sidewall portions are molded polypropylene and the fastening members are straps having hook and loop fasteners at their ends.

3. The brace of claim 1 in which the spherical are microspheres having a diameter in the range of 10 to 200 microns.

4. The brace of claim 1 in which the microspheres are plastic walled microspheres.

5. The brace of claim 1 in which the specific gravity of the composite mixture is between 0.2 and 0.5.
